# EUROPEAN PATENT SPECIFICATION

(11) **EP 1 494 659 B1**
(45) Date of publication and mention of the grant of the patent: **12.10.2005**
(21) Application number: 03726353.0
(22) Date of filing: 17.04.2003
(51) Int. Cl.: A61K 31/19, A61K 31/22

(54) **PHARMACEUTICAL COMPOSITION COMPRISING AN ALPHA-KETOALKANOIC ACID ESTER OR -AMIDE AND LACTIC ACID OR A LACTIC ACID SALT**
PHARMAZEUTISCHE ZUSAMMENSETZUNG ENTHALTEND EIN ALPHA-KETOALKANSÄUREESTER ODER -AMID UND MILCHSÄURE ODER EIN MILCHSÄURESALZ
COMPOSITION PHARMACEUTIQUE COMPRENANT UN ESTER OU UN AMIDE D'ACIDE ALPHA-CETOALCANOIQUE ET DE L'ACIDE LACTIQUE OU UN SEL D'ACIDE LACTIQUE

(30) Priority: 17.04.2002 US 373680 P
(43) Date of publication of application: 12.01.2005
(73) Proprietor: Critical Therapeutics, Inc., Lexington, MA 02421 (US)
(72) Inventor: FINK, Mitchell, P., Pittsburgh, PA 15238 (US)
(74) Representative: Luderschmidt, Schüler & Partner GbR
(86) International application number: PCT/US2003/012134
(87) International publication number: WO 2003/088955

(56) References cited:
- EP-A- 0 345 082
- WO-A-01/24793
- US-A- 4 105 783

## Description

### GOVERNMENT SUPPORT

The invention was supported, in whole or in part, by grants from the Defense Advanced Research Projects Agency (N65236-00-1-5434) and NIH grants GM58484, GM37631 and GM68481. The Government has certain rights in the invention.

### BACKGROUND OF THE INVENTION

Esters of alpha-ketoalkanoic acids such as ethyl pyruvate have been described for use in treating reperfusion injury (WO01/24793), inflammatory disorders (WO02/074301) and renal failure (WO02/081020). Notwithstanding the acceptance of esters of alpha-ketoalkanoic acids as effective therapeutic agents, esters of alpha-ketoalkanoic acids are typically unstable in aqueous solutions, presumably due to hydrolysis of the ester. For example, a sharp drop in pH from 6 to about 2 can occur in as few as four hours when such esters are dissolved in aqueous solutions. Such solutions are unsuitable for administration to patients unless used immediately after mixing. The full potential of esters of alpha-ketoalkanoic acids as therapeutic agents is unlikely to be realized without the development of pharmaceutical formulations in which the ester is stable.

### SUMMARY OF THE INVENTION

It has now been discovered esters of alpha-ketoalkanoic acids remain stable in aqueous formulations comprising lactate. For example, a Lactated Ringers Balanced Salt Solution comprising 1%, 5% and 10% by weight of ethyl pyruvate showed a relatively reduced drop in pH over a 48 hour period (Example 1). Based on this discovery, novel pharmaceutical formulations comprising esters of alpha-ketoalkanoic acids and methods of using the same in therapy are disclosed herein.

The disclosed invention is a pharmaceutical composition comprising an ester or amide of an alpha-ketoalkanoic acid and lactic acid or a pharmaceutically acceptable salt of lactic acid (i.e., lactate).

The pharmaceutical compositions of the present invention offer several advantages including high stability of alpha-ketoalkanoic acid esters compared with previously known formulations of such compounds. They are suitable for use in humas for at least 48 hours after mixing.

### DETAILED DESCRIPTION OF THE INVENTION

The present invention is a pharmacological composition comprising an aqueous solution of an ester of an alpha-ketoalkanoic acids or an amide of an alpha-ketoalkanoic acid.

In one aspect, the ester of the alpha ketoalkanoic acid used in the disclosed formulation is, for example, an ester of a C₃-C₈ straight-chained or branched alpha-ketoalkanoic acid. Examples include an ester of alpha-keto-butanoic acid, alpha-ketopentanoic acid, alpha-keto-3-methyl-butanoic acid, alpha-keto-4-methyl-pentanoic acid or alpha-keto-hexanoic acid. Pyruvic acid is preferred. A variety of groups are suitable for the ester position of the molecule, e.g., alkyl (preferably, C1-C3 alkyl), aralkyl, alkoxyalkyl of carbalkoxyalkyl. Specific examples include ethyl, propyl, butyl, carbmethoxymethyl (-CH₂COOCH₃), carbethoxymethyl (-CH₂COOCH₂CH₃), acetoxymethyl (-CH₂OC(O)CH₃), carbmethoxyethyl (-CH₂CH2COOCH₃), carbethoxyethyl (-CH₂CH₂COOCH₂CH₃), methoxymethyl (-CH₂OCH₃) and ethoxymethyl (-CH₂OCH₂CH₃). Ethyl esters are preferred. Thiolesters (e.g., wherein the thiol portion is cysteine or homocysteine), are also included. Other groups suitable for esterification of alpha-ketoalkanoic acids include dihydroxyacetone esters of formula 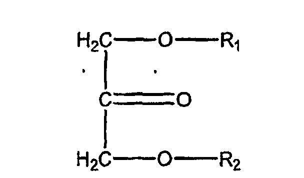 wherein R₁ is pyruvyl and R₂ is H, pyruvyl or a C1-C3 acyl, such as acetyl or propionyl;
and monosaccharide esters such as, e.g., rybosyl esters 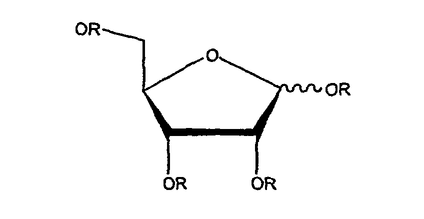 wherein each R is independently H, pyruvyl or a C1-C3 acyl, such as acetyl or propionyl, and at least one R is a pyruvyl,
or glucosyl esters 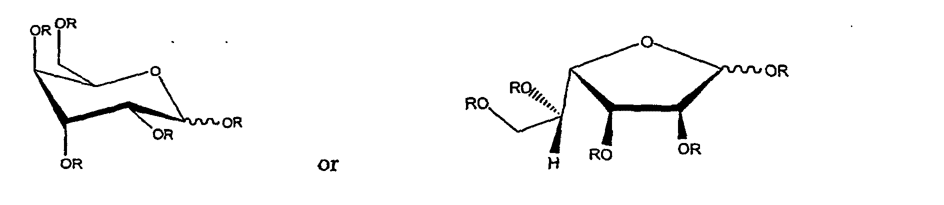 wherein each R is independently H, pyruvyl or a C1-C3 acyl, such as acetyl or propionyl, and at least one R is a pyruvyl.

In a preferred embodiment, the disclosed pharmaceutical composition comprises ethyl pyruvate, propyl pyruvate, butyl pyruvate, carbmethoxymethyl pyruvate, carbethoxymethyl pyruvate, acetoxymethyl pyruvate, carbmethoxyethyl pyruvate, carbethoxyethyl pyruvate, methoxymethyl pyruvate and ethoxymethyl pyruvate. Other preferred examples include ethyl alpha-keto-butyrate, ethyl alpha-keto-pentanoate, ethyl alpha-keto-3-methyl-butyrate, ethyl alpha-keto-4-methyl-pentanoate, or ethyl alpha-keto-hexanoate. Ethyl pyruvate is more preferred.

Suitable amides of alpha-ketoalkanoic acids for use in the disclosed formulations include compounds having the following structural formula: RCOCONR1R2. R is an alkyl group; R1 and R2 are independently -H, alkyl, aralkyl, alkoxyalkyl, carboxyalkyl or -CHR3COOH; and R3 is the side chain of a naturally occurring amino acid. Preferably, the amide of an alpha-ketoalkanoic acids is a pyruvamide.

Suitable alkyl groups include C₁-C₈ straight chained or branched alkyl group, preferably C₁-C₆ straight chained alkyl groups.

Suitable aryl groups include carbocyclic (e.g., phenyl and naphthyl) and heterocyclic (e.g., furanyl and thiophenyl) aromatic groups, preferably phenyl.

An alkoxy group is -OR4, wherein R4 is an alkyl group, as defined above. An alkoxyalkyl group is an alkyl group substituted with -OR4.

An aralkyl group is -XY, wherein X is an alkyl group and Y is an aryl group, both as defined above.

A carboxyalkyl group is an alkyl group substituted with -COOH. A carbalkoxyalkyl group is an alkyl group substituted with ester. Specific examples of ester substituents include ethyl, propyl, butyl, carbmethoxymethyl, carbethoxymethyl, acetoxymethyl, carbmethoxyethyl, carbethoxyethyl, methoxymethyl and ethoxymethyl. Ethyl esters are preferred.

The compositions of the present invention can comprise pharmaceutically acceptable salts of lactic acid at concentration from about 1 mM to about 100 mM. Example of pharmaceutically acceptable salts of lactic acid include, but are not limited to, lithium lactate, sodium lactate, potassium lactate, ammonium lactate, calcium lactate and magnesium lactate. In a preferred embodiment, sodium or potassium lactate are used.

The pharmaceutical compositions of the present invention can further include one or more of pharmaceutically acceptable salts of divalent cations such as Ca²⁺ or Mg²⁺ and monovalent cations such as Li⁺, Na⁺, K⁺, NH₄⁺ and the like. Sodium, potassium and calcium are preferable. Concentration of any one salt can vary from 0 to about 250 mM, preferably from about 10 mM to about 250 mM. Preferably, the salts are sodium chloride, potassium chloride, and calcium chloride. In a preferred embodiment, the amount of any one salt or a combination of salts is sufficient to render the composition isotonic (having physiological osmolarity of blood plasma).

The ester of the alpha-ketoalkanoic acid (e.g. pyruvate) is preferably present in the pharmaceutical formulation at a concentration from about 0.1 to about 10% by weight, preferably 2% to about 5% by weight, more preferably 2.5%-3.5% by weight.

In a preferred embodiment, the pharmaceutical composition of the present invention comprises:
(a) from about 0.1 % by weight to about 10% by weight of an ester of an alpha-ketoalkanoic acid (e.g., ethyl pyruvate); and
(b) sodium or potassium lactate at a concentration from about 1 mM to about 100 mM.
In preferred embodiments, the compositions of the present invention further comprise:
(c) from about 10 mM to about 250 mM of NaCl;
(d) from about 0.1 mM to about 25 mM of KCl;
(e) from about 0.1 mM to about 25 mM of CaCl₂.
In one preferred embodiment, the composition of the present invention comprises:
(a) about 2% by weight to about 5% by weight of ethyl pyruvate;
(b) about 50 to about 150 mM of NaCl;
(c) about 1 to about 8 mM of KCl;
(d) about 1 to about 5 mM of CaCl₂; and
(e) about 10 to about 40 mM of sodium lactate,
wherein the ingredients (a) through (e) are dissolved in an aqueous solution. More preferably, the ester in part (a) described above is present at between about 2.5% to 3.5% by weight.

One preferred example of a composition of the present invention is a Ringer's Lactate solution, which contains potassium chloride (0 to about 4 mM), sodium chloride (about 100 to about 156 mM), calcium chloride (about 2.5 mM to about 3.0 mM), and sodium lactate (about 25 mM to about 30 mM) for example, as described herein.

The pharmaceutical formulation of the present invention can be used to treat and/or ameliorate disorders such as acute renal failure and ileus. As used herein, "ileus" is a partial or complete non-mechanical obstruction of the small and/or large intestine. Ileus occurs when peristalsis, the rhythmic contraction that moves material through the bowel, stops. Ileus can be caused, for example, by manipulation of the intestines during abdominal surgery, inflammation of the peritoneum, or administration of narcotics or chemotherapeutic agents.

The pharmaceutical compositions of the present invention can further be used for reanimation and resuscication of mammals, e.g. humans, before, during and after mesenteric ischemia, mesenteric thrombus or mesenteric venous occlusion; aortic aneurism repair, coronary artery bypass, surgical treatment of arterial occlusion; hemorrhagic shock; and preservation or transplantation of organs. As used herein, "ischemia" is defined as interruption of oxygen supply, via blood flow, to an organ or to a part thereof.

The pharmaceutical compositions of the present invention can further be used to treat and/or alleviate cytokine-mediated inflammatory disorders. Such disorders include, but are not limited to, local and systemic inflammation, inflammatory bowel disease (Crohn's disease and ulcerative colitis), rheumatoid arthritis, asthma, sepsis, septic shock, inflammatory skin conditions such as psoriasis and eczema, rheumatoid arthritis, rheumatoid spondylitis, osteoarthritis, gouty arthritis and other arthritic conditions, endotoxic shock, Gram-negative shock, toxic shock syndrome, adult respiratory distress syndrome, cerebral malaria, chronic pulmonary inflammatory disease, silicosis, pulmonary sarcoidosis, bone resorption disease, reperfusion injury, graft v. host rejection, allograft rejection, fever and myalgia due to infection, cachexia secondary to infection or malignancy or secondary to AIDS, AIDS related complex, keloid and scar tissue formation.

The precise dose to be employed in a pharmaceutical composition of the present invention will depend on the route of administration, and the seriousness of the conditions, and should be decided according to the judgment of a practitioner and each patient's circumstances. Effective doses may be extrapolated from dose-response curves derived from *in vitro* or animal model test systems.

The pharmaceutical compositions of the invention can be administered through a variety of routes, for example, oral, dietary, topical, intravenous, intramuscular, or by inhalation (e.g., intrabronchial, intranasal or oral inhalation, intranasal drops) routes of administration, depending on the agent and disease or condition to be treated, using routine methods in physiologically-acceptable inert carrier substances. Other suitable methods of administration can also include rechargeable or biodegradable devices, and slow release polymeric devices. For example, the therapeutic compositions can be administered in a sustained release formulation using a biodegradable biocompatible polymer, or by on-site delivery using micelles, gels, liposomes, or a buffer solution. Preferably, the pharmaceutical composition is administered as an infusate at a concentration of, e.g., 10 mM to 200 mM, preferably 20 mM to 90 mM of the active agent, at a rate of 1 mg/kg body weight/day to 200 mg/kg body weight/day, in a buffer solution as described herein. More preferably, the pharmaceutical composition is administered as an infusate at a concentration of about 28 mM of the active agent at a dose of 100 mg/kg body weight/day to 150 mg/kg body weight/day of alpha-ketoalkanoic acid, in a buffer solution. In bolus form, the active agent can be administered at a similar dosage, e.g., 1 mg/kg body weight/day to 200 mg/kg body weight/day of active agent, where the dosage is divided into aliquots and delivered 1 to 4 times daily (for a total dosage of 1 mg/kg body weight/day to 200 mg/kg body weight/day), with the concentration of the active agent adjusted accordingly.

### EXEMPLIFICATION

### Example 1 Ethyl pyruvate is stable in Lactated Ringer's Solution over Forty-Eight Hours

Formulations comprising ethyl pyruvate at various concentrations (5, 10, and 10 % by weight) were prepared by dissolving ethyl pyruvate in either commercially available Ringer's Lactate solution (potassium chloride (0 to about 4 mM), sodium chloride (about 100 to about 156 mM), calcium chloride (about 2.5 mM to about 3.0 mM), and sodium lactate (about 25 mM to about 30 mM)) or Ringer-like solution (potassium chloride (0 to about 4 mM), sodium chloride (about 100 to about 156 mM) and calcium chloride (about 2.5 mM to about 3.0 mM)), or Intralipid™ (1 000 mL contain: purified soybean oil 100-300 g, purified egg phospholipids 12 g, glycerol anhydrous 22 g, water for injection q.s. ad 1 000 mL. pH was adjusted with sodium hydroxide to pH approximately 8. Osmolality was about 300 mOsm/kg water). Acidity of each solution was measured at 0, 5, 30 and 60 minutes, 2,4,24, and 48 hours after dissolution of ethyl pyruvate. The results are presented in Table 1. As indicated by the pH readings, ethyl pyruvate remains stable in Ringer's Lactate solution, but not in Ringer-like solution or Intralipid.

**Table 1**

| Stability of Ethyl Pyruvate in Three Different Formulation as Measured by pH Change Over Time | | | | | | | | | |
|---|---|---|---|---|---|---|---|---|---|
| | **Ringer's lactate** | | | **Ringer's solution (no lactate)** | | | **Intralipid** | | |
| **EP (%)** | **1** | **5** | **10** | **1** | **5** | **10** | **1** | **5** | **10** |
| **0 min** | 6.0 | 6.0 | 6.0 | 6.5 | 6.5 | 6.5 | 6.8 | 6.8 | 6.8 |
| **5 min** | 6.0 | 5.5 | 5.3 | 6.5 | 6.0 | 5.5 | 6.8 | 6.8 | 6.5 |
| **30 min** | 5.5 | 5.3 | 5.3 | 6.5 | 6.0 | 5.3 | 6.8 | 6.5 | 6.0 |
| **60 min** | 5.5 | 5.2 | 5.2 | 5.5 | 5.2 | 5.2 | 6.5 | 6.0 | 6.0 |
| **2 h** | 5.2 | 5.2 | 5.2 | 5.5 | 5.2 | 5.2 | 6.5 | 6.0 | 6.0 |
| **4 h** | 5.2 | 5.2 | 5.2 | 5.5 | 4.0 | 2.0 | 6.5 | 6.0 | 5.5 |
| **24 h** | 5.2 | 5.2 | 5.2 | 5.5 | 2.0 | 2.0 | 6.5 | 5.5 | 4.0 |
| **48 h** | 5.2 | 5.2 | 5.0 | 5.5 | 2.0 | 2.0 | 6.5 | 5.0 | 4.0 |

## Claims

1. A pharmaceutical composition comprising:
(a) an ester of an alpha-ketoalkanoic acid or an amide of an alpha-ketoalkanoic; and
(b) lactic acid or a pharmaceutically acceptable salt of lactic acid dissolved in aqueous solution.

2. A pharmaceutical composition comprising:
(a) an ester of an alpha-ketoalkanoic acid; and
(b) lactic acid or a pharmaceutically acceptable salt of lactic acid, dissolved in aqueous solution.

3. The pharmaceutical composition of Claim 2, wherein said ester of an alpha-ketoalkanoic acid is an ester of a C₃-C₈ straight-chained alpha-ketoalkanoic acid.

4. The pharmaceutical composition of Claim 3, wherein said ester of an alpha-ketoalkanoic acid is an ester of pyruvic acid.

5. The pharmaceutical composition of Claim 3, wherein said ester of an alpha-ketoalkanoic acid is an ethyl ester.

6. The pharmaceutical composition of Claim 3, wherein said ester of an alpha-ketoalkanoic acid is an alkyl, aralkyl, alkoxyalkyl or carbalkoxyalkyl ester.

7. The pharmaceutical composition of Claim 4, wherein said ester of pyruvic acid is selected from the group consisting of ethyl pyruvate, propyl pyruvate, carbmethoxymethyl pyruvate, acetoxymethyl pyruvate, carbethoxyethyl pyruvate, and ethoxymethyl pyruvate.

8. The pharmaceutical composition of Claim 5, wherein said ester of an alpha-ketoalkanoic acid is selected from the group consisting of ethyl alpha-keto-butyrate, ethyl alpha-keto-pentanoate, and ethyl alpha-keto-hexanoate.

9. The pharmaceutical composition of Claim 3, wherein said alpha-ketoalkanoic acid ester is a glyceryl ester, a dihydroxyacetone ester, monosaccharide ester or a thiolester.

10. The pharmaceutical composition of Claim 6 wherein said ester of an alpha-ketoalkanoic acid is a C1-C3 alkyl ester.

11. The pharmaceutical composition of Claim 10, wherein the pharmaceutical composition comprises an ester of an alpha-ketoalkanoic acid and sodium or potassium lactate.

12. The pharmaceutical composition of Claim 11 further comprising a pharmaceutically acceptable salt of Na⁺, K⁺, NH₄⁺, Mg²⁺, Ca²⁺ or a combination thereof.

13. The pharmaceutical composition of Claim 12 wherein the ester of an alpha-ketoalkanoic acid is ethyl pyruvate.

14. The pharmaceutical composition of Claim 12 wherein the pharmaceutically acceptable salts are NaCl, KCl, CaCl₂ or combinations thereof.

15. An aqueous pharmaceutical composition comprising:
(a) from 0.1% to 1.0% by weight of ethyl pyruvate; and
(b) sodium or potassium lactate at a concentration from about 1 mM to about 100 mM.

16. The pharmaceutical composition of Claim 15 further comprising:
(c) from about 10 mM to about 250 mM of NaCl;
(d) from about 0.1 mM to about 25 mM of KCl;
(e) from about 0.1 mM to about 25 mM of CaCl₂.

17. A pharmaceutical composition comprising:
(a) about 2% to about 5% by weight of ethyl pyruvate;
(b) about 50 to about 150 mM of NaCl;
(f) about 1 to about 8 mM of KCl;
(g) about 1 to about 5 mM of CaCl₂; and
(h) about 10 to about 40 mM of sodium lactate,
wherein the ingredients (a) through (e) are dissolved in an aqueous solution.

18. A pharmaceutical composition comprising from about 0.1 % to about 10% by weight of ethyl pyruvate in Ringer's Lactate solution.

19. The pharmaceutical composition of Claim 18 wherein the concentration of ethyl pyruvate is from about 2.5 % to about 3.5% by weight.

## Patentansprüche

1. Pharmazeutische Zusammensetzung, umfassend:
(a) einen Ester einer alpha Ketoalkansäure oder ein Amid einer alpha Ketoalkansäure und
(b) Milchsäure oder ein pharmazeutisch akzeptables Milchsäuresalz, das in wässriger Lösung aufgelöst ist.

2. Pharmazeutische Zusammensetzung, umfassend:
(a) einen Ester einer alpha Ketoalkansäure und
(b) Milchsäure oder ein pharmazeutisch akzeptables Milchsäuresalz, das in wässriger Lösung aufgelöst ist.

3. Pharmazeutische Zusammensetzung nach Anspruch 2, **dadurch gekennzeichnet, dass** der Ester einer alpha Ketoalkansäure ein Ester einer geradkettigen C₃-C₈ alpha Ketoalkansäure ist.

4. Pharmazeutische Zusammensetzung nach Anspruch 3, **dadurch gekennzeichnet, dass** der Ester einer alpha Ketoalkansäure ein Pyrotraubensäureester ist.

5. Pharmazeutische Zusammensetzung nach Anspruch 3, **dadurch gekennzeichnet, dass** der Ester einer alpha Ketoalkansäure ein Ethylester ist.

6. Pharmazeutische Zusammensetzung nach Anspruch 3, **dadurch gekennzeichnet, dass** der Ester einer alpha Ketoalkansäure ein Alkyl-, Aralkyl-, Alkoxyalky- oder Carbalkoxyalkylester ist.

7. Pharmazeutische Zusammensetzung nach Anspruch 4, **dadurch gekennzeichnet, dass** der Ester der Pyrotraubensäure aus der Gruppe ausgewählt ist, die aus Ethylpyruvat, Propylpyruvat, Carbmethoxymethylpyruvat, Acetoxymethylpyruvat, Carbethoxyethylpyruvat und Ethoxymethylpyruvat besteht.

8. Pharmazeutische Zusammensetzung nach Anspruch 5, **dadurch gekennzeichnet, dass** der Ester einer alpha Ketoalkansäure aus der Gruppe ausgewählt ist, die aus Ethyl-alpha-ketobutyrat, Ethyl-alpha-keto-pentanoat und Ethyl-alpha-ketohexanoat besteht.

9. Pharmazeutische Zusammensetzung nach Anspruch 3, **dadurch gekennzeichnet, dass** der alpha Ketoalkansäureester ein Glycerylester, ein Dihydroxyacetonester, ein Monosaccharidester oder ein Thiolester ist.

10. Pharmazeutische Zusammensetzung nach Anspruch 6, **dadurch gekennzeichnet, dass** der Ester einer alpha Ketoalkansäure ein C1-C3-Alkylester ist.

11. Pharmazeutische Zusammensetzung nach Anspruch 10, **dadurch gekennzeichnet, dass** die pharmazeutische Zusammensetzung einen Ester einer alpha Ketoalkansäure und Natrium- oder Kaliumlactat umfasst.

12. Pharmazeutische Zusammensetzung nach Anspruch 11, **dadurch gekennzeichnet, dass** sie weiterhin ein pharmazeutisch akzeptables Salz von Na⁺, K⁺, NH₄⁺, Mg²⁺, Ca²⁺ oder einer Kombination davon umfasst.

13. Pharmazeutische Zusammensetzung nach Anspruch 12, **dadurch gekennzeichnet, dass** der Ester einer alpha Ketoalkansäure Ethylpyruvat ist.

14. Pharmazeutische Zusammensetzung nach Anspruch 12, **dadurch gekennzeichnet, dass** die pharmazeutische akzeptablen Salze NaCl, KCl, CaCl₂ oder Kombinationen davon sind.

15. Wässrige pharmazeutische Zusammensetzung, umfassend:
(a) 0,1 Gew.-% bis 1,0 Gew.-% Ethylpyruvat und
(b) Natrium- oder Kaliumlactat mit einer Konzentration von ungefähr 1 mM bis ungefähr 100 mM.

16. Pharmazeutische Zusammensetzung nach Anspruch 15, **dadurch gekennzeichnet, dass** sie weiterhin umfasst:
(c) ungefähr 10 mM bis ungefähr 250 mM NaCl;
(d) ungefähr 0,1 mM bis ungefähr 25 mM KCl;
(e) ungefähr 0,1 mM bis ungefähr 25 mM Ca Cl₂.

17. Pharmazeutische Zusammensetzung, umfassend:
(a) ungefähr 2 Gew.-% bis ungefähr 5 Gew.-% Ethylpyruvat;
(b) ungefähr 50 bis ungefähr 150 mM NaCl;
(f) ungefähr 1 bis ungefähr 8 mM KCl;
(g) ungefähr 1 bis ungefähr 5 mM CaCl₂ und
(h) ungefähr 10 bis ungefähr 40 mM Natriumlactat,
wobei die Bestandteile (a) bis (e) in einer wässrigen Lösung aufgelöst sind.

18. Pharmazeutische Zusammensetzung, die ungefähr 0,1 Gew.-% bis ungefähr 10 Gew.-% Ethylpyruvat in Ringers Lactatlösung umfasst.

19. Pharmazeutische Zusammensetzung nach Anspruch 18, **dadurch gekennzeichnet, dass** die Konzentration von Ethylpyruvat von ungefähr 2,5 Gew.-% bis ungefähr 3,5 Gew.-% ist.

## Revendications

1. Composition pharmaceutique comprenant:
(a) un ester d'un acide α-cétoalcanoïque ou un amide d'un acide α-cétoalcanoïque; et
(b) de l'acide lactique ou un sel pharmaceutiquement acceptable d'acide lactique dissous en une solution aqueuse.

2. Composition pharmaceutique comprenant:
(a) un ester d'un acide α-cétoalcanoïque; et
(b) de l'acide lactique ou un sel pharmaceutiquement acceptable d'acide lactique, dissous en une solution aqueuse.

3. Composition pharmaceutique selon la revendication 2, dans laquelle ledit ester d'acide α-cétoalcanoïque est un ester d'un acide α-cétoalcanoïque linéaire en C₃-C₈.

4. Composition pharmaceutique selon la revendication 3, dans laquelle ledit ester d'acide α-cétoalcanoïque est un ester d'acide pyruvique.

5. Composition pharmaceutique selon la revendication 3, dans laquelle ledit ester d'acide α-cétoalcanoïque est un ester éthylique.

6. Composition pharmaceutique selon la revendication 3, dans laquelle ledit ester d'acide α-cétoalcanoïque est un ester d'alkyle, d'aralkyle, d'alcoxyalkyle ou de carbalcoxyalkyle.

7. Composition pharmaceutique selon la revendication 4, dans laquelle ledit ester d'acide pyruvique est choisi dans le groupe constitué par le pyruvate d'éthyle, le pyruvate de propyle, le pyruvate de carbométhoxyméthyle, le pyruvate d'acétoxyméthyle, le pyruvate de carbéthoxyéthyle et le pyruvate d'éthoxyméthyle.

8. Composition pharmaceutique selon la revendication 5, dans laquelle ledit ester d'acide α-cétoalcanoïque est choisi dans le groupe constitué par l'α-cétobutyrate d'éthyle, l'α-cétopentanoate d'éthyle et l'α-cétohexanoate d'éthyle.

9. Composition pharmaceutique selon la revendication 3, dans laquelle ledit ester d'acide α-cétoalcanoïque est un ester de glycéryle, un ester de dihydroxyacétone, un ester de monosaccharide ou un ester de thiol.

10. Composition pharmaceutique selon la revendication 6, dans laquelle ledit ester d'acide α-cétoalcanoïque est un ester d'alkyle en C₁-C₃.

11. Composition pharmaceutique selon la revendication 10, la composition pharmaceutique comprenant un ester d'un acide α-cétoalcanoïque et du lactate de sodium ou de potassium.

12. Composition pharmaceutique selon la revendication 11, comprenant en outre un sel pharmaceutiquement acceptable de Na⁺, K⁺, NH₄⁺, Mg²⁺, Ca²⁺ ou une de leurs combinaisons.

13. Composition pharmaceutique selon la revendication 12, dans laquelle l'ester d'acide α-cétoalcanoïque est le pyruvate d'éthyle.

14. Composition pharmaceutique selon la revendication 12, dans laquelle les sels pharmaceutiquement acceptables sont NaCl, KCl, CaCl₂ ou leurs combinaisons.

15. Composition pharmaceutique aqueuse comprenant:
(a) de 0,1 % à 1,0 % en masse de pyruvate d'éthyle; et
(b) du lactate de sodium ou de potassium à une concentration d'environ 1 mM à environ 100 mM.

16. Composition pharmaceutique selon la revendication 15, comprenant en outre:
(c) environ 10 mM à environ 250 mM de NaCl ;
(d) environ 0,1 mM à environ 25 mM de KCl ;
(e) environ 0,1 mM à environ 25 mM de CaCl₂.

17. Composition pharmaceutique comprenant :
(a) environ 2 % à environ 5 % en masse de pyruvate d'éthyle ;
(b) environ 50 mM à environ 150 mM de NaCl ;
(f) environ 1 mM à environ 8 mM de KCl ;
(g) environ 1 mM à environ 5 mM de CaCl₂ ;
(h) environ 10 à environ 40 mM de lactate de sodium,
les ingrédients (a) à (e) étant dissous dans une solution aqueuse.

18. Composition pharmaceutique comprenant d'environ 0,1 % à environ 10 % en masse de pyruvate d'éthyle dans un solution de lactate de Ringer.

19. Composition pharmaceutique selon la revendication 18, dans laquelle la concentration du pyruvate d'éthyle est d'environ 2,5 % à environ 3,5 % en masse.
